# EUROPEAN PATENT SPECIFICATION

(11) **EP 2 579 818 B1**
(45) Date of publication and mention of the grant of the patent: **16.04.2014**
(21) Application number: 12743576.6
(22) Date of filing: 06.07.2012
(51) Int. Cl.: A61F 2/30, A61F 2/38

(54) **APPARATUS FOR AUGMENTING THE POSTERIOR ASPECT OF A FEMORAL KNEE JOINT PROSTHESIS COMPONENT**
VORRICHTUNG ZUR VERBESSERUNG DER HINTERANSICHT EINER OBERSCHENKEL-KNIEGELENKPROTHESENKOMPONENTE
APPAREIL POUR AUGMENTER L'ASPECT POSTÉRIEUR D'UN COMPOSANT FÉMORAL DE PROTHÈSE D'ARTICULATION DE GENOU

(30) Priority: 07.07.2011 US 201161505310 P
(43) Date of publication of application: 17.04.2013
(60) Divisional application: 14158181.9
(73) Proprietor: Zimmer, Inc., Warsaw IN 46580 (US)
(72) Inventor: METZGER, Dianne, S., Huntington, IN 46750 (US); VAUGHAN, Ian, R., Fort Wayne, IN 46814 (US); TODD, Dwight, T., Columbia City, IN 46725 (US)
(74) Representative: Mays, Julie
(86) International application number: PCT/US2012/045825
(87) International publication number: WO 2013/006823

(56) References cited:
- EP-A1- 0 378 928
- EP-A2- 0 745 361
- US-A- 5 370 693
- US-A1- 2005 149 052

## Description

### BACKGROUND

### 1. Field of the Disclosure.

The present disclosure relates generally to a femoral prosthesis component augment. More particularly, the present disclosure relates to apparatus for augmenting the posterior aspect of a femoral knee joint prosthesis component. The closest prior art is document US 2005/149052 A1, which defines the preamble of claim 1.

### 2. Description of the Related Art.

Knee replacement surgery methods and knee joint prostheses are known in the art. A typical total knee joint prosthesis includes a tibial component that is attached to a proximal portion of a tibia, and a femoral component that is attached to a distal portion of a femur. The femoral component rides on the exposed surface of the tibia component, replicating knee movement and providing articulation similar to the natural, anatomical articulation of the knee joint. When knee replacement surgery is performed, an incision is made to expose the knee joint in order to enable removal of both the proximal portion of the tibia and the distal portion of the femur, which creates surfaces upon which the tibial component and the femoral component of the knee prosthesis can be attached.

In certain situations, the surgeon must remove more of the distal femur than desired, because of disease or trauma. Consequently, there are times when a femoral component selected by a surgeon to provide good joint kinematics once knee arthroplasty is completed has a distance spanning the anterior bone contacting surface to the posterior bone contacting surface that is greater than a distance spanning an anterior prepared distal femur surface to a posterior prepared distal femur surface. In these situations, femoral prosthesis augments can be utilized to provide structural support and to prevent gaps between the distal femur and the femoral component of the knee prosthesis installed on the distal femur.

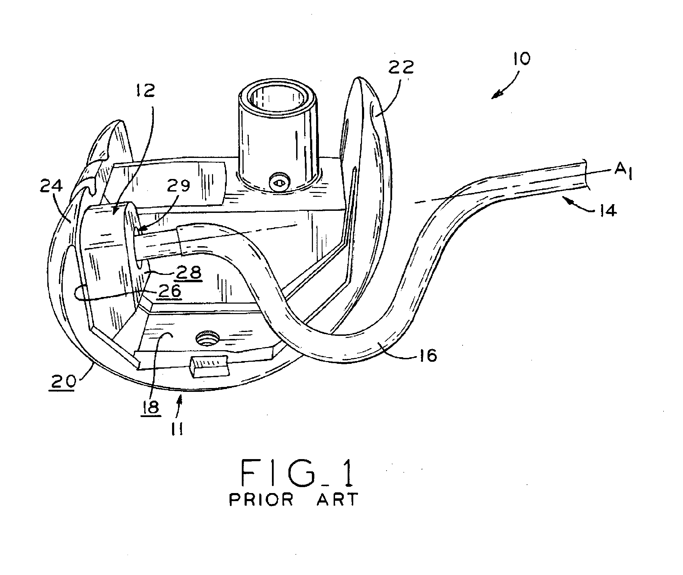

An existing femoral knee prosthesis system including a femoral augment, and method of use are shown in the "Zimmer® NexGen® Trabecular Metal Augments, Abbreviated Surgical Technique" brochure, copyright 2004, 2006, published by Zimmer, Inc. Fig. 1 is representative of a femoral knee prosthesis system utilized with the above surgical technique. Referring to Fig. 1, femoral knee prosthesis system 10 includes femoral component 11, femoral posterior augment 12, and offset drive shaft 16.

Femoral component 11 generally includes bone contacting surface 18, opposing articular surface 20, anterior flange 22, and posterior side 24 opposite anterior flange 22. Further, femoral component 11 has a bore (not shown) located in posterior side 24. The bore (not shown) located in posterior side 24 has a longitudinal axis A₁ which intersects anterior flange 22.

Femoral posterior augment 12 generally includes femoral component contacting surface 26, opposing outer surface 28, and augment cavity 29 spanning femoral component contacting surface 26 and outer surface 28. With augment 12 positioned adjacent posterior side 24 of femoral component 11 and augment cavity 29 aligned with the bore (not shown) located in posterior side 24 of femoral component 11, augment cavity 29 has a longitudinal axis that is collinear with longitudinal axis A₁ of the bore (not shown) located in posterior side 24 and which also intersects anterior flange 22.

Referring to Fig. 1, proximal end 14 of offset drive shaft 16 is operably connected to a torque wrench (not shown) and used to tighten a fastener (not shown) into the bore (not shown) located in posterior side 24 of femoral component 11 to secure femoral posterior augment 12 to posterior side 24 of femoral component 11. Femoral knee prosthesis system 10 requires offset drive shaft 16 to avoid anterior flange 22 of femoral component 11 when securing augment 12 to femoral component 11. However, offset drive shaft 16 of femoral knee prosthesis system 10 cannot be rotated through 360 degrees because anterior flange 22 provides a physical barrier preventing drive shaft 16 from continuous rotation. In femoral knee prosthesis system 10, a straight drive shaft cannot be utilized to tighten a fastener to secure posterior augment 12 to posterior side 24 of femoral component 11 because anterior flange 22 provides a physical barrier which blocks a straight drive shaft from being used.

### SUMMARY

The present invention is defined in claim 1 and provides a femoral component having an anterior flange and a condyle opposite the anterior flange. The condyle can be augmented by a femoral posterior augment. The femoral posterior augment is securable to the condyle by a fastener which is actuated to a securement position by a rotatable driver which does not contact the anterior flange through rotation of the driver sufficient to actuate the fastener to secure the femoral augment to the condyle.

In one embodiment, the present invention provides a femoral component having a condyle with a bore formed therein, the bore having a longitudinal axis which does not intersect the anterior flange of the femoral component, and a femoral posterior augment having a cavity complementary to the bore of the femoral component. The femoral component and the femoral posterior augment in accordance with embodiments of the present invention allows a fastener to be placed in both the cavity of the femoral posterior augment and the bore of the femoral component and allows a tool, such as a torque wrench, having a straight driver to be used to secure the femoral posterior augment to a posterior facet of the femoral component. Because the longitudinal axis of the bore does not intersect the anterior flange, an offset driver is not needed to secure the posterior augment to the posterior facet of the femoral component because the anterior flange does not have to be avoided when securing the posterior augment to the posterior facet of the femoral component. The straight driver that may be used in accordance with one form of the present invention has a linear longitudinal axis and is capable of transferring an impaction force from one end to another end of the driver along its longitudinal axis and is further capable of transferring torque from the one end to the other end about its longitudinal axis.

In one embodiment, the fastener of the present invention may include a set screw which traverses a cavity of the posterior augment and the bore of the femoral component to secure the posterior augment to the condyle of the femoral component. In another embodiment, the fastener of the present invention may include a set screw and an expansion collet. In one embodiment, the expansion collet may extend from the augment and with the expansion collet of the augment aligned in the bore of the femoral component, the set screw may traverse the cavity of the posterior augment and the bore of the femoral component to engage the expansion collet with a securement feature of the condyle to secure the posterior augment to the condyle. In an alternative embodiment, the expansion collet may extend from the condyle of the femoral component and with the expansion collet of the condyle aligned in the cavity of the posterior augment, the set screw may traverse the cavity of the posterior augment and the bore of the femoral component to engage the expansion collet with a securement feature of the posterior augment to secure the posterior augment to the condyle.

In one embodiment, the present invention provides a femoral knee prosthesis component including an anterior flange, the anterior flange having an anterior flange bone contacting surface comprising an anterior facet, a condyle extending from the anterior flange, the condyle having a condyle bone contacting surface comprising a facet opposing the anterior facet, the condyle having a condyle bore formed therein, the condyle bore defining a condyle bore longitudinal axis that intersects the facet and does not intersect the anterior flange. In an embodiment of the present invention, the condyle bore longitudinal axis intersects the facet at an intersection point, the intersection point positioned such that an axis perpendicular to the facet and intersecting the intersection point may have a minimum spacing from the anterior flange of no more than 8 mm. In further embodiments the minimum space may be no more than 5 mm. In one embodiment, the condyle bore longitudinal axis may not be perpendicular to the condyle bone contacting surface. In one form of the present invention, the femoral knee prosthesis component may be provided in combination with a posterior augment for securement to the condyle and a fastener operable to secure the augment to the condyle. In further embodiments, the combination may include a driver, the driver comprising a handle and a drive shaft extending from the handle and terminating in a drive end, the drive shaft defining a longitudinal axis, the drive end mateable with the fastener, the drive shaft defining a drive shaft radius from the longitudinal axis of the drive shaft to a perimeter extent of the drive shaft, with the drive shaft positioned to drive the fastener into an engaged position to secure the augment to the condyle, the driver is rotatable through a rotation about the longitudinal axis of the drive shaft sufficient to actuate the fastener to secure the augment to the condyle and the longitudinal axis of the drive shaft is positioned a minimum distance from the anterior flange throughout the rotation about the longitudinal axis of the drive shaft sufficient to actuate the driver to secure the augment to the condyle, the minimum distance at least equal to the drive shaft radius, whereby the drive shaft does not contact the anterior flange through the rotation about the longitudinal axis of the drive shaft sufficient to actuate the fastener to secure the augment to the condyle.

The present invention, in a further form thereof, comprises a femoral knee prosthesis component including an anterior flange, the anterior flange having an anterior flange bone contacting surface comprising an anterior facet, a condyle extending from the anterior flange, the condyle having a condyle bone contacting surface comprising a facet opposing the anterior facet, the condyle having a condyle bore formed therein, the condyle bore defining a condyle bore longitudinal axis that intersects the facet and does not intersect the anterior flange, an augment for securement to the condyle, the augment including an augment bore formed therethrough, and a fastener sized and shaped to traverse the augment bore and secure the augment to the condyle.

The present invention, in another form thereof, comprises, in combination, a femoral knee prosthesis component including an anterior flange, the anterior flange having an anterior flange bone contacting surface. The femoral knee prosthesis component of this form of the present invention further includes a condyle extending from the anterior flange, the condyle having a condyle bone contacting surface, the condyle bone contacting surface opposing the anterior flange bone contacting surface, the condyle having a condyle bore formed therein, the condyle bore defining a condyle bore longitudinal axis which does not intersect the anterior flange. The combination of this form of the present invention may further include a posterior augment for securement to the condyle, the posterior augment having an augment bore formed therethrough, the augment bore defining an augment bore longitudinal axis, the condyle including a securement feature extending into the condyle bore. The combination of this form of the present invention may additionally include a fastener, the fastener traversing the augment bore and the condyle bore to engage the securement feature and secure the augment to the condyle, with the condyle bore longitudinal axis aligned with the augment bore longitudinal axis. The combination of this form of the present invention may further include a driver including a handle and a drive shaft extending from the handle and terminating in a drive end, the drive end matable with the fastener, the drive shaft defining a drive shaft radius to a perimeter extent of the drive shaft, the condyle bore longitudinal axis spaced a minimum distance from the anterior flange bone contacting surface, the minimum distance at least equal to the drive shaft radius. In one exemplary embodiment, the minimum distance may be 4 mm. In one embodiment, the anterior flange bone contacting surface comprises an anterior facet and the minimum distance is measured in a plane including the anterior facet.

The present invention, in a further form thereof, comprises a femoral prosthesis system including a femoral knee prosthesis component having an anterior flange, the anterior flange having an anterior flange bone contacting surface, and a condyle extending from the anterior flange, the condyle having a condyle bone contacting surface opposing the anterior flange bone contacting surface. The femoral prosthesis system of this form of the present invention may further include an augment for securement to the condyle. The femoral prosthesis system of this form of the present invention may further include a fastener operable to secure the augment to the condyle, the fastener defining a fastener longitudinal axis, with the fastener positioned to secure the augment to the condyle, the fastener longitudinal axis does not intersect the anterior flange.

The present invention, in another form thereof, comprises, in combination, a femoral prosthesis system including a femoral knee prosthesis component including an anterior flange, the anterior flange having an anterior flange bone contacting surface, and a condyle extending from the anterior flange, the condyle having a condyle bone contacting surface, the condyle bone contacting surface opposing the anterior flange bone contacting surface. The combination of this form of the present invention may further include an augment for securement to the condyle. The combination of this form of the present invention may additionally include a fastener operable to secure the augment to the condyle. The combination of this form of the present invention may further include a driver including a handle and a drive shaft extending from the handle and terminating in a drive end, the drive shaft defining a longitudinal axis, the drive end matable with the fastener, the drive shaft defining a drive shaft radius from the longitudinal axis of the drive shaft to a perimeter extent of the drive shaft, with the drive shaft positioned to drive the fastener into an engaged position to secure the augment to the condyle, the driver is rotatable through a rotation about the longitudinal axis of the drive shaft sufficient to actuate the fastener to secure the posterior augment to the condyle and the longitudinal axis of the drive shaft is positioned a minimum distance from the anterior flange throughout the rotation about the longitudinal axis of the drive shaft sufficient to actuate the fastener to secure the posterior augment to the condyle, the minimum distance at least equal to the drive shaft radius, whereby the drive shaft does not contact the anterior flange through the rotation about the longitudinal axis of the drive shaft sufficient to actuate the fastener to secure the posterior augment to the condyle. In one exemplary embodiment, the minimum distance may be 4 mm.

The present invention may be manufactured by a method of manufacturing a femoral prosthesis component useable with a posterior augment, the method including: forming a femoral prosthesis component of a biologically compatible material, the femoral prosthesis component including an anterior flange having an anterior flange bone contacting surface; and a condyle extending from the anterior flange, the condyle having a condyle bone contacting surface, the condyle bone contacting surface opposing the anterior flange bone contacting surface; selecting a rotary forming tool, the rotary forming tool having a drive shaft with a drive shaft periphery and a longitudinal axis, the drive shaft defining a drive shaft radius to the drive shaft periphery; positioning the rotary forming tool such that the longitudinal axis of the drive shaft of the rotary forming tool intersects the condyle bone contacting surface of the condyle at an intersection point and is oblique to the condyle bone contacting surface and the longitudinal axis of the drive shaft of the rotary forming tool is spaced a minimum distance from the anterior flange bone contacting surface, the minimum distance at least equal to the drive shaft radius, wherein, with the rotary forming tool longitudinal axis intersecting the intersection point, the anterior flange presents a physical barrier preventing rotary forming tool from being positioned perpendicular to the condyle bone contacting surface; and, with the rotary forming tool positioned as defined in the positioning step, actuating the rotary forming tool to form a bore through the condyle bone contacting surface and into the condyle.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
Fig. 1 is a perspective view of a prior art femoral knee prosthesis system including a femoral component, a femoral posterior augment, and an offset driver;
Fig. 2 is a perspective view of a femoral component in accordance with the present disclosure;
Fig. 3 is a perspective view of a femoral posterior augment in accordance with the present disclosure;
Fig. 4 is an exploded perspective view of a femoral knee prosthesis system in accordance with the present disclosure;
Fig. 5 is a partial sectional, plan view of a femoral knee prosthesis system in accordance with the present disclosure with a posterior side of a femoral component and a femoral posterior augment shown in section;
Fig. 6A is a fragmentary, cross-sectional view of the posterior side of the femoral component of Fig. 5 and the femoral posterior augment of Fig. 5, illustrating collet fingers of the femoral posterior augment in a non-expanded position;
Fig. 6B is a fragmentary, cross-sectional view of the posterior side of the femoral component of Fig. 5 and the femoral posterior augment of Fig. 5, illustrating collet fingers of the femoral posterior augment in an expanded position;
Fig. 7A is a perspective view of a femoral knee prosthesis system including a femoral distal augment and a femoral posterior augment in accordance with the present disclosure;
Fig. 7B is a cross-sectional view taken along line 7B-7B of Fig. 7A;
Fig. 8 is a partial sectional, plan view of a femoral knee prosthesis system in accordance with another exemplary embodiment of the present disclosure with a posterior side of a femoral component and a femoral posterior augment shown in section;
Fig. 9A is a fragmentary, cross-sectional view of a posterior side of a femoral component and a femoral posterior augment of a femoral knee prosthesis system in accordance with another exemplary embodiment of the present disclosure, illustrating collet fingers of the femoral component in a non-expanded position;
Fig. 9B is a fragmentary, cross-sectional view of the posterior side of the femoral component of Fig. 9A and the femoral posterior augment of the Fig. 9A, illustrating collet fingers of the femoral component in an expanded position; and
Fig. 10 is a perspective view illustrating a method of manufacturing a femoral prosthesis component useable with a posterior augment in accordance with the present disclosure.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION

In the following discussion, "proximal" refers to a direction generally toward the heart of a patient, and "distal" refers to the opposite direction of proximal, i.e., away from the heart of a patient. As used herein, "anterior" refers to a direction generally toward the front of a patient, and "posterior" refers to the opposite direction of anterior, i.e., toward the back of a patient. Further, as used herein, "medial" refers to a direction generally toward the middle of a patient, and "lateral" refers to the opposite direction of medial, i.e., toward the side of a patient. While the exemplary embodiments detailed herein are shown and described with regard to a left knee, it will be appreciated that the present disclosure is equally applicable to a right knee configuration.

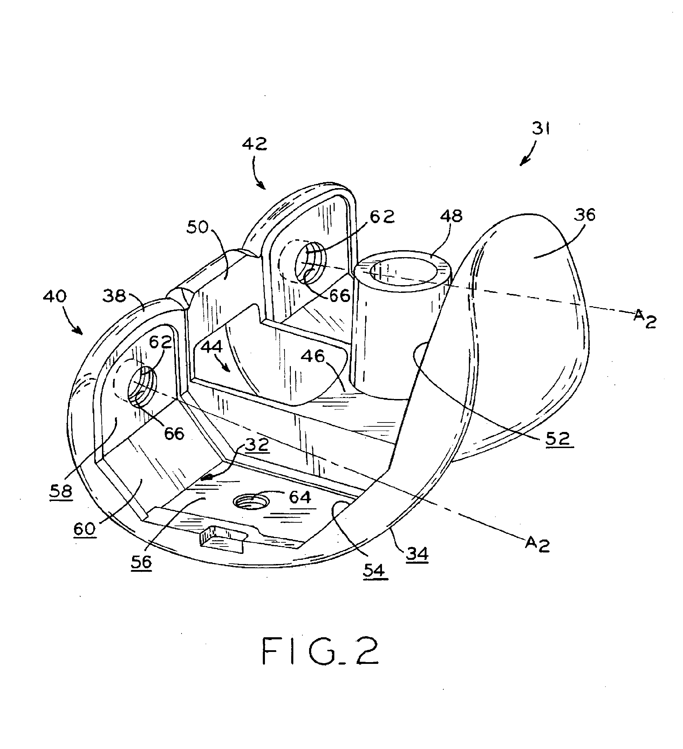

Fig. 2 illustrates a femoral component according to an exemplary embodiment of the present disclosure. Femoral component 31 of the present disclosure is adapted to mount to a distal femur. In the exemplary embodiment of Fig. 2, femoral component 31 is a posterior stabilized femoral component, though it is contemplated that other femoral components may be utilized in accordance with the present disclosure such as femoral components which cooperate to form a cruciate retaining prosthesis or a knee prosthesis having an intermediate level of constraint between a cruciate retaining and posterior stabilized prosthesis. Femoral component 31 may also be made available in a variety of shapes and sizes to accommodate a variety of differing knee physiologies.

Femoral component 31 generally includes bone contacting interior surface 32 and opposing articular exterior surface 34, each extending between anterior flange 36 and posterior side 38. Bone contacting surface 32 is adapted to affix femoral component 31 to a distal portion of a femur, such as with bone cement and/or porous bone-ingrowth material. Femoral component 31 also includes medial condyle 40 and lateral condyle 42, with intercondylar fossa 44 formed between condyles 40, 42. Bone contacting surface 32 of condyles 40, 42 at posterior side 38 opposes anterior flange 36. Condyles 40, 42 are joined by intercondylar box 46. Stem 48 extends proximally from intercondylar box 46. Articular exterior surface 34 is disposed generally opposite bone contacting interior surface 32, and is comprised of the exterior surfaces of medial and lateral condyles 40, 42 as well as the exterior surface of anterior flange 36. Femoral component 31 also includes femoral cam 50 formed at posterior side 38. Femoral cam 50 spans medial and lateral condyles 40, 42.

In a posterior stabilized femoral component, such as femoral component 31, cam 50 cooperates with a spine (not shown) formed in a tibial component (not shown) to guide or constrain motion within certain predefined boundaries. Posterior stabilized prostheses are appropriate where the posterior cruciate ligament (PCL) is torn or otherwise damaged, or where the PCL is resected during surgery.

Bone contacting interior surface 32 of femoral component 31 is adapted to mate with a resected articular surface of a distal femur (not shown) and includes anterior facet 52, anterior chamfer facet 54, distal facet 56, posterior facet 58, and posterior chamfer facet 60. Anterior facet 52, anterior chamfer facet 54, posterior facet 58, and posterior chamfer facet 60 correspond to the cuts made to a distal end of a femur to allow implantation of a femoral prosthesis component, i.e., an anterior cut, an anterior chamfer cut, a posterior cut, and a posterior chamfer cut. Anterior facet 52 and posterior facet 58 diverge from each other in a distal to proximal direction to facilitate a surgical technique in which femoral component 11 can be implanted on a femur in a distal to proximal direction. In one exemplary embodiment, anterior facet 52 diverges from posterior facet 58 such that a plane containing anterior facet 52 forms an angle of 1-5° with the plane containing posterior facet 58. Femoral component 31 may be part of a provisional prosthesis system or a final prosthesis system, i.e., femoral component 31 is the final femoral component implanted to a resected distal femur.

Referring to Fig. 2, femoral component 31 includes bores 62 formed in posterior facet 58 on medial condyle 40 and lateral condyle 42. While described with reference to posterior facet 58, the securement feature of the present disclosure may also be formed in posterior chamfer facet 60. Generally, the present invention is employed with a facet of the femoral component that opposes another feature of the femoral component such that the feature presents a barrier to a tool accessing the securement feature along a trajectory perpendicular to the facet with which the securement feature is associated. A bore wall 66 defines each bore 62 having longitudinal axis A₂. Bores 62 are blind bores, i.e., they do not extend completely from posterior facet 58 through to articular exterior surface 34. Longitudinal axis A₂ of bore 62 does not intersect anterior flange 36. In the embodiment illustrated in Fig. 2, posterior facet 58 and anterior facet 52 are generally parallel and longitudinal axis A₂ is not perpendicular to posterior facet 58. As will be understood by the drawings and the description herein, longitudinal axis A₂ represents an imaginary line extending beyond the limits of bore 62. In this embodiment, longitudinal axis A₂ of bore 62 is oblique to posterior facet 58 and anterior facet 52, i.e., longitudinal axis A₂ of bore 62 is neither parallel nor perpendicular to posterior facet 58 and anterior facet 52. In other embodiments, posterior facet 58 of femoral component 31 may be canted relative to anterior facet 52, i.e., posterior facet 58 may not be parallel to anterior facet 52.

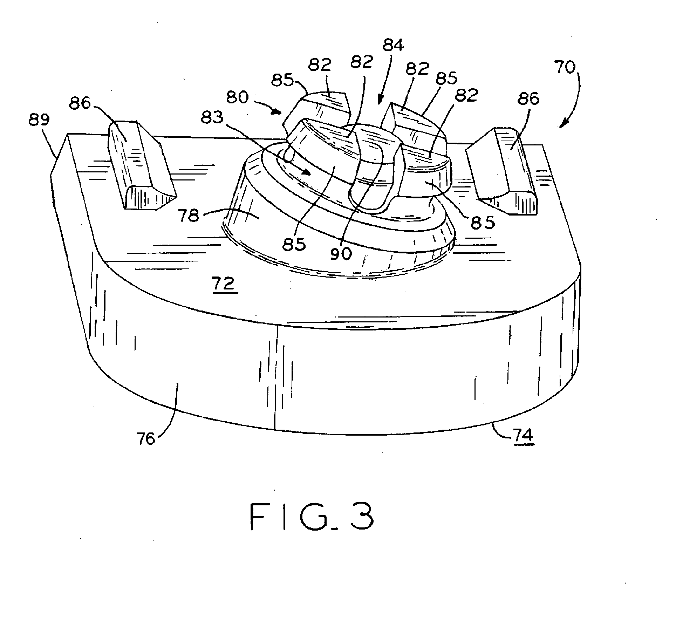

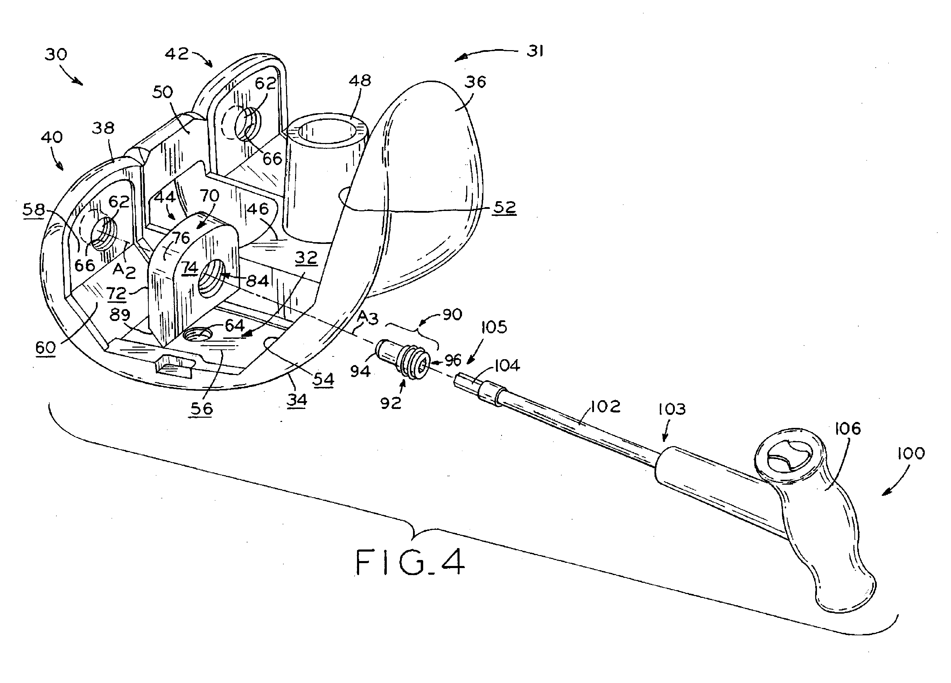

Advantageously, femoral component 31 allows a tool, such as a torque wrench, having a straight driver (such as straight drive shaft 102 shown in Figs. 4, 5, and 8) to be used to secure a femoral posterior augment (such as posterior augment 70 shown in Figs. 3, 4, 5, and 8) to posterior facet 58 of femoral component 31. A straight driver such as straight drive shaft 102 includes a linear longitudinal axis from one end (such as first end 103 shown in Figs. 4, 5, and 8) to another end (such as second end 105 shown in Figs. 4, 5, and 8). Straight drive shaft 102 is capable of transferring an impaction force from first end 103 (shown in Figs. 4, 5, and 8) to second end 105 (shown in Figs. 4, 5, and 8) along longitudinal axis A₂ and is capable of imparting torque from first end 103 to second end 105 about longitudinal axis A₂.

By having bores 62 with longitudinal axes A₂ which do not intersect anterior flange 36, an offset driver (such as offset drive shaft 16 shown in Fig. 1) is not needed to secure posterior augment 70 to posterior facet 58 of femoral component 31 because anterior flange 36 does not have to be avoided when securing posterior augment 70 to posterior facet 58 of femoral component 31.

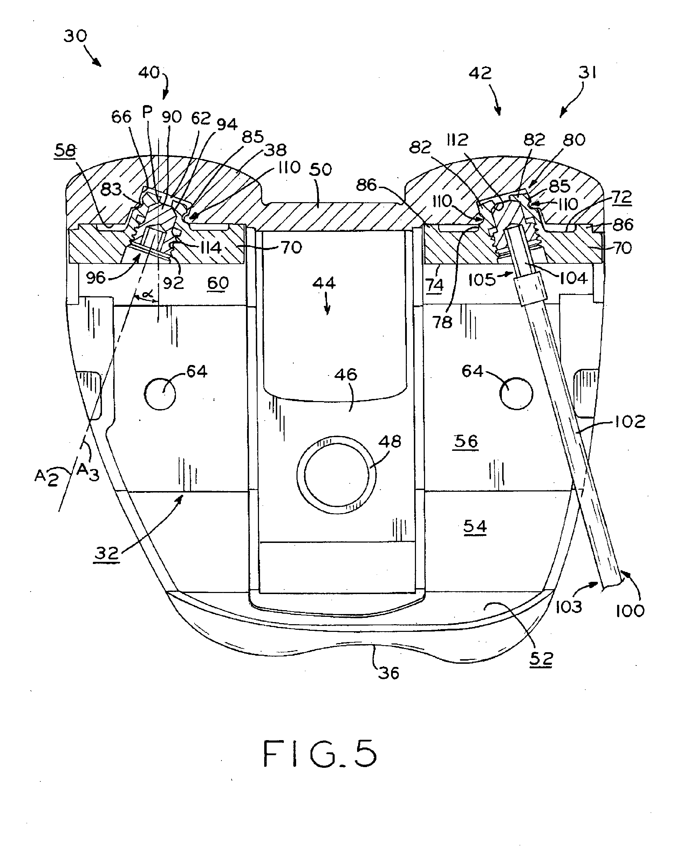

Fig. 5 illustrates perpendicular P which intersects longitudinal axis A₂ of bore 62 at a plane containing posterior facet 58. Perpendicular P is perpendicular to posterior facet 58 and, in certain embodiments, intersects anterior flange 36. In alternative embodiments of the present invention, perpendicular P has a minimum spacing from anterior flange 36 of no more than 8 mm. In further embodiments, perpendicular P has a minimum spacing from anterior flange 36 of no more than 5 mm. Defining perpendicular P as having a minimum spacing from anterior flange 36 of no more than 8 mm or no more than 5 mm is meant to be inclusive of embodiments in which perpendicular P intersects anterior flange 36, i.e., has a minimum spacing of zero. That is, "not more than" is inclusive of any value including to or less than the stated value. In this context "minimum spacing" denotes the shortest distance between perpendicular P and anterior flange 36. Through this document phrases such as "minimum spacing" and "minimum distance" denote the shortest distance between two items, such as a physical structure of the prosthesis and an imaginary line or axis. In one exemplary embodiment, condyle bore longitudinal axis A₂ forms an angle α with perpendicular P in the range of 7-27°. In an exemplary embodiment the angle α is in the range of 12-22°. In a further exemplary embodiment the angle α equals 17°. In the illustrated embodiment, angle α is measured in a transverse anatomic plane. In certain embodiments, angle α will be measured in a plane parallel to distal facet 56.

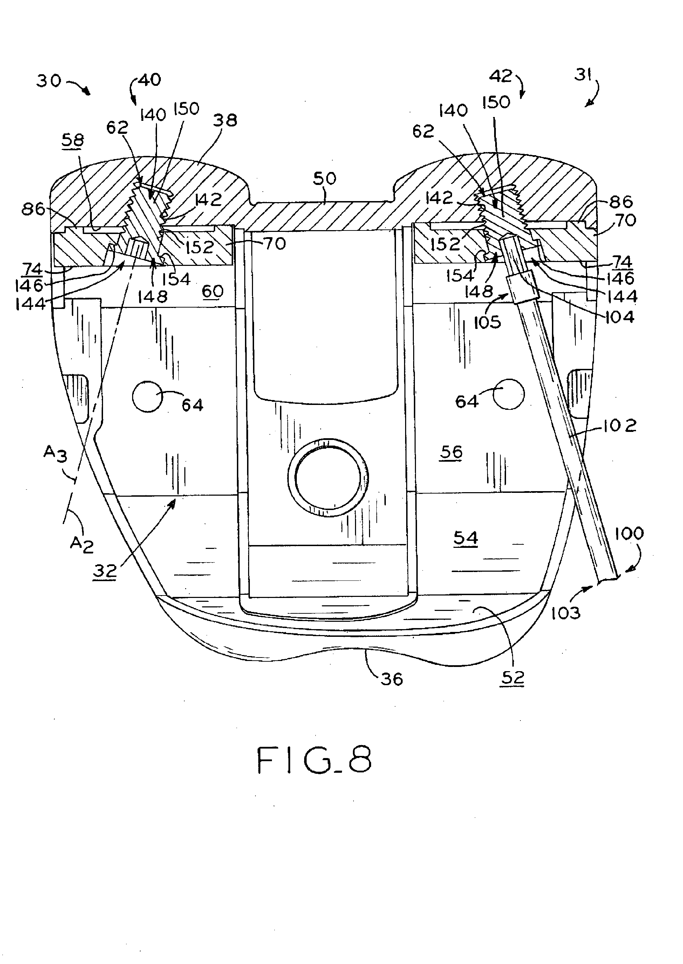

Referring to Figs. 5 and 8, longitudinal axis A₂ of bore 62 does not intersect anterior flange 36 and longitudinal axis A₂ of bore 62 is spaced from anterior flange 36 a minimum distance. The minimum distance is at least as great as the extent of straight drive shaft 102 from its longitudinal axis to allow straight drive shaft 102 to avoid anterior flange 36 when straight drive shaft 102 is used to effect securement of posterior augment 70 to posterior side 38 of femoral component 31. In an exemplary embodiment, the drive shaft is a cylindrical drive shaft of having a radius of about 4 mm. In such an embodiment, the minimum distance between longitudinal axis A₂ of bore 62 and anterior flange 36 is 4 mm. In the embodiment illustrated in Figs. 2, 5 and 8, the minimum distance is measured in a plane including anterior facet 52. With set screw 90 or fastener 140 in position to be secured to attach posterior augment 70 to posterior side 38 of femoral component 31, the portion of drive shaft 102 adjacent anterior flange 36 during the act of securement, i.e., until set screw 90 or fastener 140 is fastened in bore 62, is spaced a distance that is at least as great as the extent of drive shaft 102 from its longitudinal axis. In one embodiment, the entire drive shaft 102 is spaced from anterior flange 36 a distance that is at least as great as the extent of drive shaft 102 from its longitudinal axis.

In the embodiment illustrated in Figs. 5 and 8, straight drive shaft 102 has a cylindrical cross-sectional shape. In such an embodiment, longitudinal axis A₂ of bore 62 is spaced from anterior flange 36 by at least the radius of straight drive shaft 102. In other embodiments, drive shaft 102 can have multi-sided polygon cross-sectional shapes, such as square or rectangular cross-sectional shapes. In such embodiments, a perimeter extent of drive shaft 102 is the radially outward portion farthest from the longitudinal axis of drive shaft 102, and the cross-section of drive shaft 102 defines a drive shaft radius to the perimeter extent of drive shaft 102. In these embodiments, longitudinal axis A₂ of bore 62 is spaced from anterior flange 36 by at least the radius of drive shaft 102. In alternative embodiments, drive shaft 102 can have an irregular shape, e.g., drive shaft 102 could have an inconsistent cross-section along its length and/or have a shaft center through certain cross-sections that is not always coincident with the longitudinal axis of the drive shaft. In embodiments including drive shafts having irregular shapes, the drive shaft could have perimeter extents a varying distance from the longitudinal axis of the drive shaft. In such embodiments, longitudinal axis A₂ of bore 62 is spaced from anterior flange 36 by at least the distance between the longitudinal axis of the drive shaft and the perimeter extent of the drive shaft which will be adjacent to anterior flange 36 in use to secure posterior augment 70 to posterior side 38 of femoral component 31. In this way, drive shaft 102 will, in use, be rotatable about its longitudinal axis through 360 degrees of rotation without engaging anterior flange 36. In certain embodiments, rotation of drive shaft through 360° of rotation will not be required to actuate a fastener to secure a posterior augment to the condyle. In any event, the embodiments of the present disclosure will allow sufficient rotation of a driver to actuate a fastener to secure a posterior augment to the posterior condyle of a femoral prosthesis.

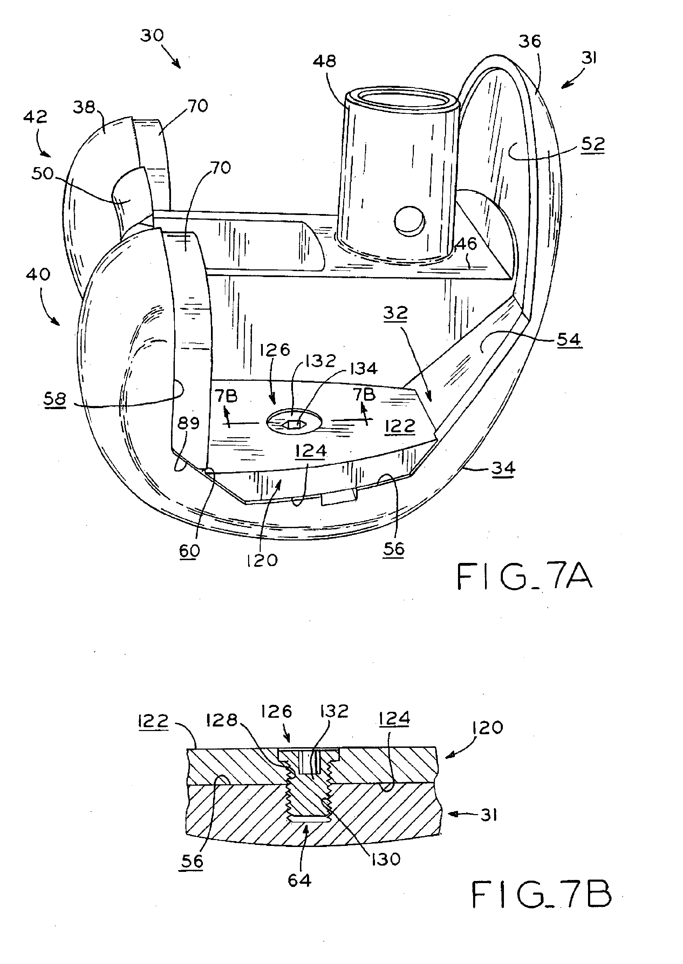

Referring to Figs. 2 and 7A, femoral component 31 also includes distal bore 64 located in distal facet 56 for receiving a fastener to secure a femoral distal augment (such as distal augment 120 shown in Fig. 7A) to distal facet 56 as will be discussed in more detail later.

Femoral component 31 may be constructed of any biocompatible ceramic or metal, including, but not limited to, titanium, a titanium alloy, cobalt chromium, cobalt chromium molybdenum, porous tantalum, or a combination of these materials, for example. Femoral component 31 may include a highly porous biomaterial useful as a bone substitute and as cell and tissue receptive material. A highly porous biomaterial may have a porosity as low as 55%, 65%, or 75% or as high as 80%, 85%, or 90%. An example of such a material is produced using Trabecular Metal^{™} technology generally available from Zimmer, Inc., of Warsaw, Indiana. Trabecular Metal^{™} is a registered trademark of Zimmer, Inc. Such a material may be formed from a reticulated vitreous carbon foam substrate which is infiltrated and coated with a biocompatible metal, such as tantalum, by a chemical vapor deposition ("CVD") process in the manner disclosed in detail in U.S. Patent No. 5,282,861 to Kaplan. In addition to tantalum, other metals such as niobium, or alloys of tantalum and niobium with one another or with other metals may also be used.

Generally, the porous tantalum structure includes a large plurality of ligaments defining open spaces therebetween, with each ligament generally including a carbon core covered by a thin film of metal such as tantalum, for example. The open spaces between the ligaments form a matrix of continuous channels having no dead ends, such that growth of cancellous bone through the porous tantalum structure is uninhibited. The porous tantalum may include up to 75%, 85%, or more void space therein. Thus, porous tantalum is a lightweight, strong porous structure which is substantially uniform and consistent in composition, and closely resembles the structure of natural cancellous bone, thereby providing a matrix into which cancellous bone may grow to provide fixation of femoral component 31 to the patient's bone.

The porous tantalum structure may be made in a variety of densities in order to selectively tailor the structure for particular applications. In particular, as discussed in the above-incorporated U.S. Patent No. 5,282,861, the porous tantalum may be fabricated to virtually any desired porosity and pore size, and can thus be matched with the surrounding natural bone in order to provide an improved matrix for bone ingrowth and mineralization.

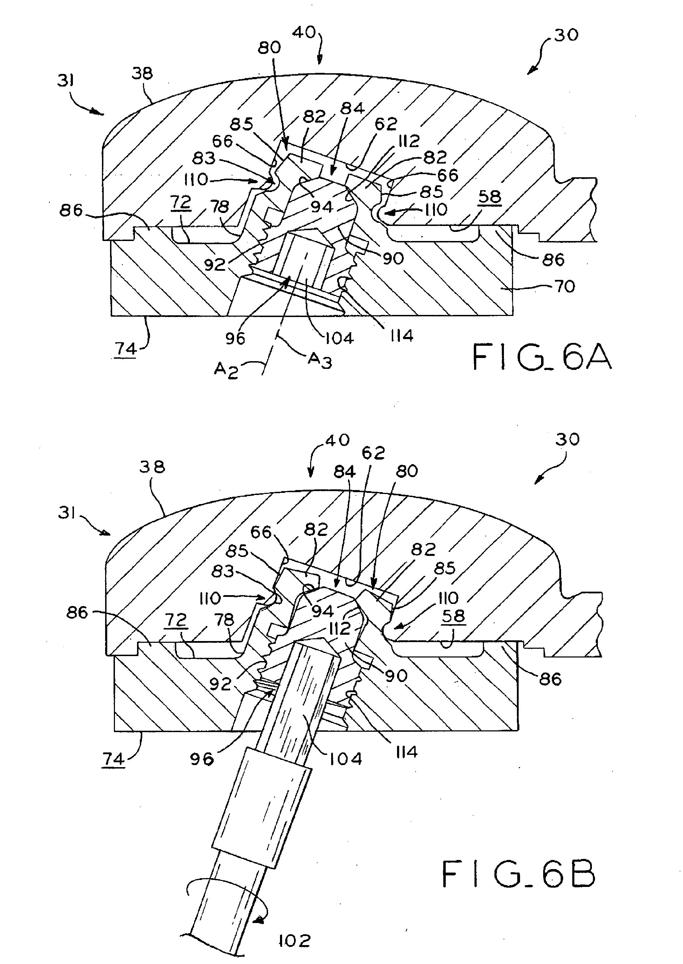

Fig. 3 illustrates features of femoral posterior augment 70 according to an exemplary embodiment of the present disclosure. Posterior augment 70 provides structural support in areas of bone loss of a distal femur and prevents gaps between the distal femur and posterior facet 58 of femoral component 31 installed on the distal femur. Posterior augment 70 generally includes femoral component contacting surface 72, opposing bone contacting surface 74, peripheral wall 76 spanning femoral component contacting surface 72 and bone contacting surface 74, boss 78, and expansion collet 80. Boss 78 extends at an angle from femoral component contacting surface 72 and includes expansion collet 80 at a terminal end thereof. The attachment of posterior augment 70 to femoral component 31 using expansion collet 80 will be discussed in more detail later. Expansion collet 80 generally includes four collet fingers 82 located around a periphery of expansion collet 80. Annular groove 83 is located between collet fingers 82 and boss 78. Collet fingers 82 include tapered exterior portion 85 and tapered interior portion 112 (shown in Figs. 6A and 6B).

Posterior augment 70 also includes augment cavity 84 spanning augment bone contacting surface 74 and the extent of collet fingers 82. Augment cavity 84 includes augment threaded interior portion 114 (shown in Figs. 6A and 6B), which, in use, receives a threaded fastener such as set screw 90. Set screw 90 will be discussed in more detail later. With augment 70 positioned adjacent bore 62 of posterior facet 58 of femoral component 31 such that augment cavity 84 is aligned with bore 62, longitudinal axis A₃ (shown in Figs. 4 and 5) of augment cavity 84 is collinear with longitudinal axis A₂ of bore 62 and does not intersect anterior flange 36.

Referring to Fig. 3, posterior augment 70 further includes two augment protuberances 86 extending from femoral component contacting surface 72. One augment protuberance 86 is located on each side of boss 78 and protuberances 86 can be used to properly index posterior augment 70 relative to posterior facet 58 of femoral component 31. In alternative embodiments, protuberances 86 can have a continuous surface which can fit with an undercut surface of posterior facet 58 of femoral component 31. Referring to Figs. 3 and 4, posterior augment 70 includes beveled edge 89. Beveled edge 89 and femoral component contacting surface 72 mimic the contour of posterior facet 58 and posterior chamfer facet 60 of femoral component 31 to allow for a close fit of femoral component contacting surface 72 of augment 70 to posterior facet 58 of femoral component 31 and beveled edge 89 of augment 70 to posterior chamfer facet 60 of femoral component 31.

Posterior augment 70 may be constructed of any bio-compatible ceramic or metal, including, but not limited to, titanium, a titanium alloy, cobalt chromium, cobalt chromium molybdenum, or porous tantalum, or a combination of the materials, for example. In certain embodiments, the augments of the present disclosure can be made of a Tivanium™ alloy (Ti-6Al-4V) generally available from Zimmer, Inc., of Warsaw, Indiana. Tivanium^{™} is a registered trademark of Zimmer, Inc. Posterior augment 70 may also be constructed in whole or in part of a highly porous biomaterial such as a material produced using Trabecular Metal^{™} technology as described above.

Similar to femoral component 31, posterior augment 70 may be part of a provisional prosthesis system or a final prosthesis system. In certain embodiments, posterior augment 70 will be disposable. For example, in a provisional prosthesis system, if posterior augment 70 is deformed in use, posterior augment 70 could be disposed of after its use as a trial.

Figs. 4-6B illustrate attachment of posterior augment 70 to posterior side 38 of femoral component 31. As shown in Fig. 4, a surgical kit in accordance with the present disclosure includes femoral component 31, posterior augment 70, set screw 90, and torque wrench 100 having straight drive shaft 102. Referring to Fig. 4, set screw 90 generally includes threaded portion 92, tapered portion 94, and internal female socket 96. Set screw 90 may be constructed of any biocompatible ceramic or metal, including, but not limited to, titanium or a titanium alloy, such as Tivanium™ available from Zimmer, Inc., for example. Torque wrench 100 generally includes straight drive shaft 102, socket head 104, and handle 106 (Fig. 4). Referring to Fig. 5-6B, protrusion 110 extends from bore wall 66 into bore 62, collet fingers 82 include tapered interior portion 112, and posterior augment 70 includes threaded interior portion 114.

Straight drive shaft 102 is operably connected to torque wrench 100 and is used to secure posterior augment 70 onto femoral component 31. Advantageously, because longitudinal axis A₂ of bore 62 of femoral component 31 does not intersect anterior flange 36, straight drive shaft 102 can be used to attach posterior augment 70 onto posterior side 38 of femoral component 31.

Posterior augment 70 is mounted on posterior side 38 of femoral component 31 such that femoral component contacting surface 72 is positioned adjacent posterior facet 58 of femoral component 31 and augment protuberances 86 contact posterior facet 58 (shown in Figs. 6A and 6B). Once posterior augment 70 is properly aligned with posterior facet 58 such that augment cavity 84 is aligned with bore 62, longitudinal axis A₃ (shown in Figs. 4 and 5) of augment cavity 84 is collinear with longitudinal axis A₂ of bore 62 and does not intersect anterior flange 36.

With cavity 84 of posterior augment 70 positioned adjacent bore 62, expansion collet 80 of posterior augment 70 is inserted into bore 62 such that fingers 82 of expansion collet 80 extend beyond posterior facet 58 of femoral component 31 and into bore 62. Referring to Figs. 5-6B, when collet fingers 82 are inserted into bore 62 of femoral component 31, tapered exterior portion 85 of collet fingers 82 slide over and past protrusion 110 in bore 62 such that collet fingers 82 are located past protrusion 110 and protrusion 110 occupies annular groove 83. Fig. 6A illustrates collet fingers 82 in a non-expanded position, with collet fingers 82 slid past protrusion 110 and annular groove 83 positioned adjacent protrusion 110. Next, set screw 90 can be positioned in augment cavity 84.

Once collet fingers 82 are in the position shown in Fig. 6A and set screw 90 is positioned in augment cavity 84, socket head 104 of straight drive shaft 102 can be inserted into internal female socket 96 of set screw 90, and set screw 90 can be tightened by rotating straight drive shaft 102 and torque wrench 100 using handle 106 (Fig. 4). As set screw 90 is threaded into augment cavity 84, tapered portion 94 of set screw 90 cooperates with tapered interior portion 112 of collet fingers 82 and pushes collet fingers 82 outward until exterior portion 85 of collet fingers 82 contacts bore wall 66 of bore 62 and locks collet fingers 82 over protrusion 110 as shown in Fig. 6B. This configuration ensures that with collet fingers 82 mechanically locked over protrusion 110, posterior augment 70 is secured to posterior facet 58 of femoral component 31, such that, significant relative movement between augment 70 and femoral component 31 is prevented.

In an alternate embodiment, referring to Fig. 8, fastener 140 is utilized to attach posterior augment 70 to posterior side 38 of femoral component 31. As shown in Fig. 8, in such an embodiment, bore 62 includes threaded bore portion 142 and posterior augment 70 includes counterbore 144 forming augment shoulder 146. Fastener 140 generally includes head 148 and shank 150 having threaded portion 152. The transition between head 148 and shank 150 define fastener shoulder 154. Fastener shoulder 154 engages augment shoulder 146 in counterbore 144 of posterior augment 70 to draw posterior augment 70 into tight engagement with femoral component 31 as fastener 140 is threaded into threaded bore portion 142. Augment shoulder 146 of posterior augment 70 is recessed sufficiently below augment bone contacting surface 74 of posterior augment 70 such that head 148 of fastener 140 does not protrude above the plane of augment bone contacting surface 74 when fastener 140 is fully tightened. In the alternate embodiment illustrated in Fig. 8, straight drive shaft 102 can be used to tighten fastener 140 to secure posterior augment 70 onto posterior side 38 of femoral component 31 because longitudinal axis A₂ of bore 62 of femoral component 31 does not intersect anterior flange 36.

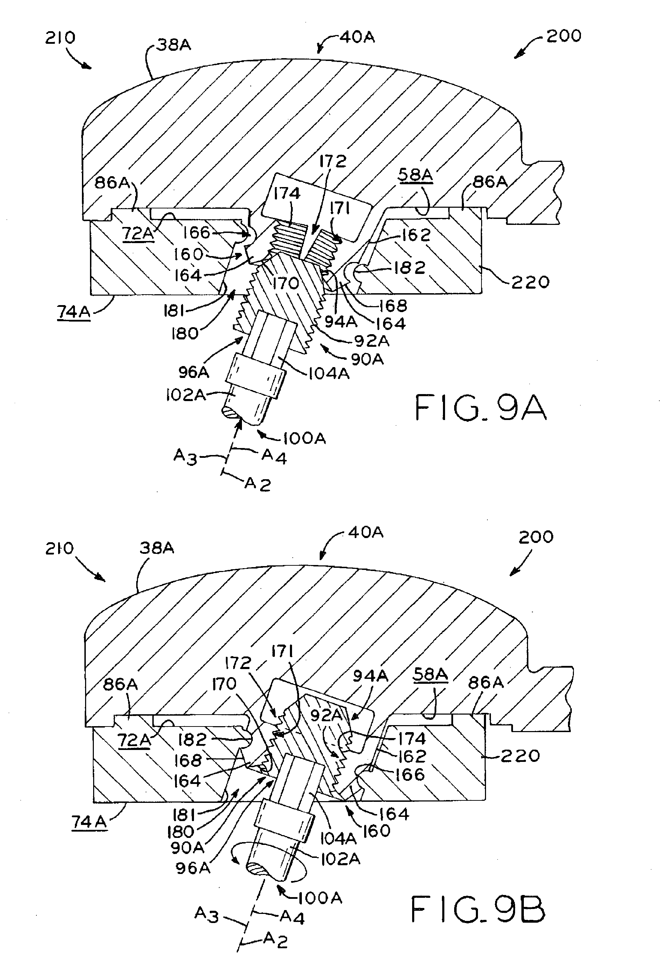

Figs. 9A and 9B illustrate another exemplary embodiment. This embodiment of the present disclosure includes similar features to the embodiment illustrated in Figs. 2-6B. These features are denoted with the same reference number used to identify the corresponding feature in the previous embodiment followed by the letter A. For the sake of brevity, these similar components will not all be discussed in conjunction with the embodiment illustrated in Figs. 9A and 9B.

Referring to Figs. 9A and 9B, femoral knee prosthesis system 200 includes femoral component 210, femoral posterior augment 220, set screw 90A, and torque wrench 100A. In this embodiment, expansion collet 160 extends from condyle 40A of femoral component 210 and condyle boss 162 extends at an angle from posterior facet 58A of femoral component 210 and includes expansion collet 160 at a terminal end thereof. In one embodiment, expansion collet 160 is formed integral to posterior facet 58A of condyle 40A. Expansion collet 160 generally includes collet fingers 164 located around a periphery of expansion collet 160. Annular groove 166 is located between collet fingers 164 and condyle boss 162. Collet fingers 164 include tapered exterior portion 168 and tapered interior portion 170. Expansion collet 160 and condyle boss 162 also include condyle bore wall 171 defining condyle bore 172 having longitudinal axis A₂. Condyle bore 172 spans the extent of collet fingers 164 and condyle boss 162. Longitudinal axis A₂ of condyle bore 172 does not intersect anterior flange 36 (Figs. 2 and 5). Referring to Figs. 9A and 9B, condyle bore 172 penetrates posterior facet 58A of femoral component 210. In other embodiments, no portion of condyle bore 172 penetrates posterior facet 58 of femoral component 210. Condyle bore 172 includes threaded interior portion 174, which, in use, receives a threaded fastener such as set screw 90A.

Referring to Figs. 9A and 9B, posterior augment 220 includes interior bore wall 181 defining augment bore 180 having longitudinal axis A₃. Augment bore 180 spans femoral component contacting surface 72A and bone contacting surface 74A. Augment bore 180 also includes augment protrusion 182 extending from bore wall 181 into augment bore 180. Posterior augment 220 is mounted to condyle 40A on posterior side 38A of femoral component 210 such that femoral component contacting surface 72A is positioned adjacent posterior facet 58A of femoral component 210 and augment protuberances 86A contact posterior facet 58A. Once posterior augment 220 is properly aligned with posterior facet 58A, expansion collet 160 of condyle 40A is inserted into augment bore 180 such that fingers 164 of expansion collet 160 extend beyond augment protrusion 182 of posterior augment 220 as shown in Figs. 9A. With augment bore 180 of posterior augment 220 receiving expansion collet 160, longitudinal axis A₃ of augment bore 180 does not intersect anterior flange 36 (Fig. 5).

When collet fingers 164 are inserted into augment bore 180 of posterior augment 220, tapered exterior portion 168 of collet fingers 164 slide over and past augment protrusion 182 in augment bore 180 such that collet fingers 164 are located past augment protrusion 182 and augment protrusion 182 occupies annular groove 166. Fig. 9A illustrates collet fingers 164 in an unexpanded position, with collet fingers 164 slid past augment protrusion 182 and annular groove 166 positioned adjacent augment protrusion 182. Next, set screw 90A can be positioned in augment bore 180 and condyle bore 172. Set screw 90A defines fastener longitudinal axis A₄ and with set screw 90A positioned to secure posterior augment 220 to condyle 40A of femoral component 210, fastener longitudinal axis A₄ does not intersect anterior flange 36 (Fig. 5).

Once collet fingers 164 are in the position shown in Fig. 9A and set screw 90A positioned in augment bore 180 and condyle bore 172, socket head 104A of straight drive shaft 102A can be inserted into internal female socket 96A of set screw 90A, and set screw 90A can be tightened by rotating straight drive shaft 102A of torque wrench 100A using handle 106 (Fig. 4). As set screw 90A is threaded into condyle bore 172, tapered portion 94A of set screw 90A cooperates with tapered interior portion 170 of collet fingers 164 and pushes collet fingers 164 outward until exterior portion 168 of collet fingers 164 contact bore wall 181 of augment bore 180 and lock collet fingers 164 over augment protrusion 182 as shown in Fig. 9B. This configuration ensures that with collet fingers 164 mechanically locked over augment protrusion 182, posterior augment 220 is secured to posterior facet 58A of femoral component 210, such that, significant relative movement between augment 220 and femoral component 210 is prevented.

Referring to Figs. 7A and 7B, femoral knee prosthesis system 30 may include distal augment 120 secured to distal facet 56 of femoral component 31. Distal augment 120 provides structural support in areas of bone loss of a distal femur and prevents gaps between the distal femur and distal facet 56 of femoral component 31 installed on the distal femur. Distal augment 120 generally includes proximal surface 122, opposing distal surface 124, and distal augment cavity 126 spanning proximal surface 122 and distal surface 124. Distal augment cavity 126 includes internally threaded portion 128.

To secure distal augment 120 to distal facet 56 of femoral component 31, distal surface 124 of distal augment 120 is positioned atop distal facet 56 such that distal augment cavity 126 is positioned adjacent to distal bore 64 (shown in Fig. 2) located on distal facet 56 of femoral component 31. Distal bore 64 includes internally threaded bore portion 130. Upon properly positioning distal augment 120 to distal facet 56 and inserting fastener 132 having female socket 134 in distal augment cavity 126, a tool having a hexagonal cross-section can be inserted in female socket 134 of threaded fastener 132. The tool having a hexagonal cross-section can then be rotated to tighten threaded fastener 132 into threaded bore portion 130 to secure distal augment 120 to distal facet 56 of femoral component 31. Because no portion of femoral component 31 opposes distal facet 56, a variety of drivers including those having a straight drive shaft may be utilized to secure threaded fastener 132 in place as described above.

Distal augment 120 may be constructed of any biocompatible ceramic or metal, including, but not limited to, titanium or a titanium alloy, for example. Similar to posterior augment 70, distal augment 120 may also be constructed of a Tivanium^{™} material generally available from Zimmer, Inc. Distal augment 120 may also be constructed in whole or in part of a highly porous biomaterial such as a material produced using Trabecular Metal^{™} technology as described above.

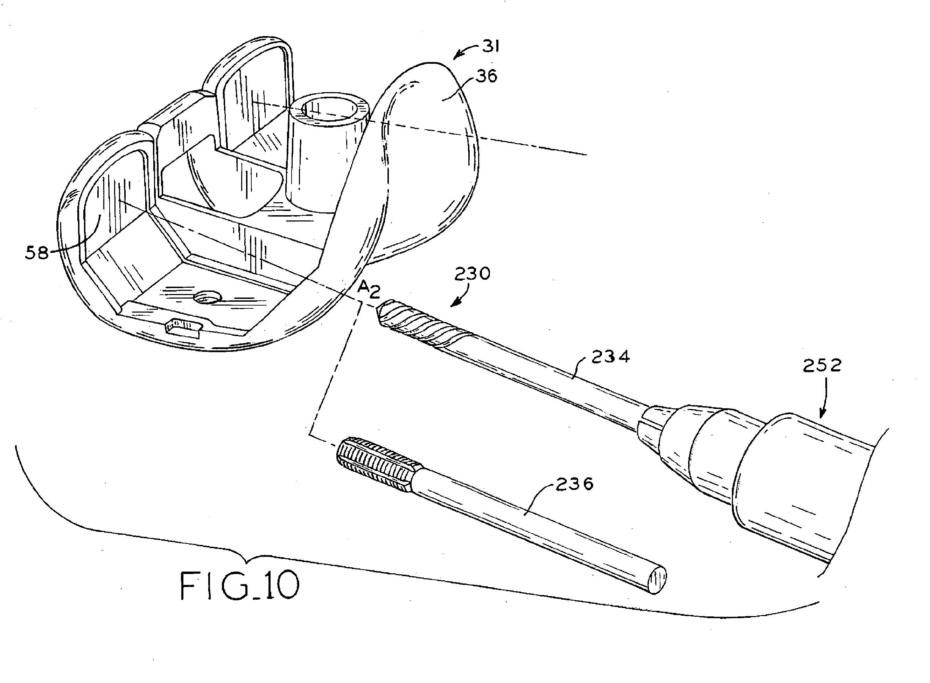

Referring to Fig. 10, bore 62 (Fig. 2) of femoral component 31 in accordance with the present disclosure may be produced using drill bit 230 to drill bore 62 in posterior facet 58 of femoral component 31. Because longitudinal axis A₂ of bore 62 does not intersect anterior flange 36, drill bit 230 can be positioned along a trajectory collinear with longitudinal axis A₂ to avoid anterior flange 36 and drill bore 62 in posterior facet 58. Advantageously, bore 62 of femoral component 31 is less costly to manufacture than conventional femoral knee prosthesis systems such as the one illustrated in Fig. 1. Referring to Fig. 1, femoral component 11 of conventional femoral knee prosthesis system 10 would not be able to have a bore (not shown) drilled in posterior side 24 using a drill bit because a drill bit would be unable to be positioned along a trajectory collinear with longitudinal axis A₁ because anterior flange 22 would block the drill bit from such a position. Thus, a more expensive and time consuming manufacturing technique such as electrical discharge machining (EDM) would be required to produce a bore in posterior side 24 of femoral component 11.

Referring to Fig. 2, during a manufacturing procedure to drill bore 62 in posterior facet 58 of femoral component 31, a location for bore 62 is determined in posterior facet 58 of femoral component 31, bore 62 having bore longitudinal axis A₂ that does not intersect anterior flange 36. Next, drill bit 230 (Fig. 10) having a drill bit width is selected for a rotary forming tool such as drill 232. Drive shaft 234 of the rotary forming tool includes a drive shaft periphery and a longitudinal axis and drive shaft 234 defines a drive shaft radius to the drive shaft periphery. Drive shaft 234 of the rotary forming tool is then positioned along a trajectory collinear with bore longitudinal axis A₂ such that with drill bit 230 positioned adjacent posterior facet 58 of femoral component 31, the longitudinal axis of drive shaft 234 does not intersect anterior flange 36 and is spaced from anterior flange 36 a minimum distance. The minimum distance is at least equal to the drive shaft radius to allow the drive shaft of the rotary forming tool to avoid anterior flange 36 when the drive shaft and the drill bit are used to form a bore through posterior facet 58 of femoral component 31. In one embodiment, the minimum distance is measured in a plane including anterior facet 52 (shown in Fig. 2). Finally, bore 62 is created by drilling a bore in posterior facet 58 of femoral component 31 using drill bit 230 positioned as described above. After bore 62 is formed, tap 236 may be utilized (e.g., together with drill 232) to thread bore 62. The drive shaft of tap 236 is sized so that it is spaced from anterior flange 36 in the same way that drive shaft 234 is spaced from anterior flange 36.

While this disclosure has been described as having exemplary designs, the application is intended to cover any variations, or adaptations of the disclosure using its general principles and to cover such departures from the present disclosure as come within known or customary practice in the art to which this disclosure pertains and which fall within the scope of the invention as defined by the appended claims.

## Claims

1. A femoral knee prosthesis (31) component, comprising:
an anterior flange (36), said anterior flange (36) having an anterior flange bone contacting surface comprising an anterior facet; and
a condyle (40,42) extending from said anterior flange (36), said condyle having a condyle bone contacting surface (32) comprising a facet (58)(60) opposing said anterior facet (36), said condyle (40,42) having a condyle bore (62) formed therein, said condyle bore (62) defining a condyle bore longitudinal axis (A2) that intersects said facet (58)(60) and does not intersect said anterior flange (36).

2. The femoral knee prosthesis component of Claim 1, wherein said condyle bore longitudinal axis (A2) intersects said facet (58)(60) at an intersection point, said intersection point positioned such that an axis (P) perpendicular to said facet (58,60) and intersecting said intersection point has a minimum spacing from said anterior flange (36) of no more than 8 mm.

3. The femoral knee prosthesis component of Claim 1, wherein said condyle bore longitudinal axis (A2) intersects said facet (58)(60) at an intersection point, said intersection point positioned such that an axis perpendicular to said facet (58)(60) and intersecting said intersection point has a minimum spacing from said anterior flange (36) of no more than 5 mm.

4. The femoral knee prosthesis component of Claim 1, wherein said facet (58)(60) comprises a posterior chamfer facet (58).

5. The femoral knee prosthesis component of Claim 1, wherein said facet (58,60) comprises a posterior chamfer facet (60).

6. The femoral knee prosthesis component of Claim 1, wherein said condyle bore longitudinal axis (A2) is spaced a minimum distance of 4 mm from said anterior flange (36).

7. The femoral knee prosthesis component of Claim 1, wherein said condyle bore longitudinal axis (A2) is oblique to said condyle bone contacting surface (32).

8. The femoral knee prosthesis of Claim 1, wherein said facet (58,60) is oriented relative to said anterior facet (36) such that said axis (P) perpendicular to said facet (58,60) and intersecting said intersection point intersects said anterior facet (36).

9. The femoral knee prosthesis component of Claim 1, wherein the condyle bore longitudinal axis (A₂) forms an angle of 7-27° with a line (P) perpendicular to the facet (58,60).

10. The femoral knee prosthesis component of Claim 1, wherein the femoral knee prosthesis component comprises a femoral knee prosthesis bone contacting surface (32) comprising the anterior facet (36) and further comprising a posterior facet (58), an anterior chamfer facet (54), a distal facet (56) and a posterior chamfer facet (60), the anterior chamfer facet (54) spanning the anterior facet (36) and the distal facet (56), the posterior chamfer facet (60) spanning the distal facet (56) and the posterior facet (58).

11. The femoral knee prosthesis component of Claim 4, wherein the posterior facet diverges proximally from the anterior facet.

12. The femoral knee prosthesis component of Claim 13, wherein a plane containing the posterior facet forms an angle of 1-5° with a plane containing the anterior facet.

13. The femoral knee prosthesis component of Claim 1, further comprising:
a posterior augment (70) for securement to said condyle (40,42); and
a fastener (90) operable to secure said posterior augment (70) to said condyle (40,42).

14. The femoral knee prosthesis component of Claim 13, wherein said posterior augment (70) includes an augment bore formed therethrough, and wherein said fastener (90) comprises a threaded fastener, said threaded fastener (90) sized and shaped to be alignable with said condyle bore (62) longitudinal axis (A2) and securable relative to said posterior augment (70) and the femoral knee prosthesis component by traversing said augment bore and said condyle bore (62) to engage said posterior augment (70) and said condyle (40,42), and secure said posterior augment (70) to said condyle (40,42).

15. The femoral knee prosthesis component of Claim 13, wherein said fastener (90) defines a longitudinal axis and a peripheral extent and wherein said fastener further defines a fastener radius to said peripheral extent, said condyle bore longitudinal axis spaced a minimum distance from said anterior flange bone contacting surface, said minimum distance at least equal to said fastener radius.

## Patentansprüche

1. Knieprothesen-Oberschenkelkomponente (31), umfassend:
einen vorderen Rand (36), wobei der vordere Rand (36) eine Knochenkontaktfläche des vorderen Rands aufweist, die eine vordere Facette umfasst; und
einen Kondylus (40, 42), der von dem vorderen Rand (36) aus verläuft, wobei der Kondylus eine Knochenkontaktfläche (32) des Kondylus aufweist, die eine Facette (58)(60) gegenüber der vorderen Facette (36) umfasst, wobei der Kondylus (40, 42) eine darin ausgebildete Kondylusöffnung (62) aufweist, wobei die Kondylusöffnung (62) eine Kondylusöffnung-Längsachse (A2) definiert, die die Facette (58)(60) schneidet und nicht den vorderen Rand (36) schneidet.

2. Knieprothesen-Oberschenkelkomponente nach Anspruch 1, wobei die Kondylusöffnung-Längsachse (A2) die Facette (58)(60) in einem Schnittpunkt schneidet, wobei der Schnittpunkt so angeordnet ist, dass eine den Schnittpunkt schneidende Achse (P) senkrecht zu der Facette (58, 60) einen Mindestabstand zu dem vorderen Rand (36) von nicht mehr als 8 mm aufweist.

3. Knieprothesen-Oberschenkelkomponente nach Anspruch 1, wobei die Kondylusöffnung-Längsachse (A2) die Facette (58)(60) in einem Schnittpunkt schneidet, wobei der Schnittpunkt so angeordnet ist, dass eine den Schnittpunkt schneidende Achse senkrecht zu der Facette (58)(60) einen Mindestabstand zu dem vorderen Rand (36) von nicht mehr als 5 mm aufweist.

4. Knieprothesen-Oberschenkelkomponente nach Anspruch 1, wobei die Facette (58)(60) eine hintere abgeschrägte Facette (58) umfasst.

5. Knieprothesen-Oberschenkelkomponente nach Anspruch 1, wobei die Facette (58)(60) eine hintere abgeschrägte Facette (60) umfasst.

6. Knieprothesen-Oberschenkelkomponente nach Anspruch 1, wobei die Kondylusöffnung-Längsachse (A2) einen Mindestabstand von 4 mm zu dem vorderen Rand (36) aufweist.

7. Knieprothesen-Oberschenkelkomponente nach Anspruch 1, wobei die Kondylusöffnung-Längsachse (A2) zu der Knochenkontaktfläche (32) des Kondylus geneigt ist.

8. Femorale Knieprothese nach Anspruch 1, wobei die Facette (58, 60) bezogen auf die vordere Facette (36) so ausgerichtet ist, dass die den Schnittpunkt schneidende Achse (P) senkrecht zu der Facette (58, 60) die vordere Facette (36) schneidet.

9. Knieprothesen-Oberschenkelkomponente nach Anspruch 1, wobei die Kondylusöffnung-Längsachse (A2) einen Winkel von 7 bis 27° mit einer Linie (P) senkrecht zu der Facette (58, 60) bildet.

10. Knieprothesen-Oberschenkelkomponente nach Anspruch 1, wobei die Knieprothesen-Oberschenkelkomponente eine Knochenkontaktfläche (32) der femoralen Knieprothese umfasst, die die vordere Facette (36) umfasst und ferner eine hintere Facette (58), eine vordere abgeschrägte Facette (54), eine distale Facette (56) und eine hintere abgeschrägte Facette (60) umfasst, wobei die vordere abgeschrägte Facette (54) eine Brücke zwischen der vorderen Facette (36) und der distalen Facette (56) bildet und die hintere abgeschrägte Facette (60) eine Brücke zwischen der distalen Facette (56) und der hinteren Facette (58) bildet.

11. Knieprothesen-Oberschenkelkomponente nach Anspruch 4, wobei sich die hintere Facette proximal von der vorderen Facette weg neigt.

12. Knieprothesen-Oberschenkelkomponente nach Anspruch 13, wobei eine die hintere Facette enthaltende Ebene einen Winkel von 1 bis 5° mit einer die vordere Facette enthaltenden Ebene bildet.

13. Knieprothesen-Oberschenkelkomponente nach Anspruch 1, ferner umfassend:
eine hintere Verstärkung (70) zur Befestigung an dem Kondylus (40, 42); und
ein Befestigungselement (90), das so einsetzbar ist, dass es die hintere Verstärkung (70) an dem Kondylus (40, 42) befestigt.

14. Knieprothesen-Oberschenkelkomponente nach Anspruch 13, wobei die hintere Verstärkung (70) eine dort hindurch ausgebildete Verstärkungsöffnung aufweist und wobei das Befestigungselement (90) ein Befestigungselement mit Gewinde umfasst, wobei das Befestigungselement (90) mit Gewinde so bemessen und geformt ist, dass es nach der Längsachse (A2) der Kondylusöffnung (62) ausrichtbar ist und bezogen auf die hintere Verstärkung (70) und die Knieprothesen-Oberschenkelkomponente befestigbar ist, indem es die Verstärkungsöffnung und die Kondylusöffnung (62) durchquert und in die hintere Verstärkung (70) und den Kondylus (40, 42) eingreift und die hintere Verstärkung (70) an dem Kondylus (40, 42) befestigt.

15. Knieprothesen-Oberschenkelkomponente nach Anspruch 13, wobei das Befestigungselement (90) eine Längsachse und eine Umfangsausdehnung definiert und wobei das Befestigungselement ferner einen Befestigungselementradius zu der Umfangsausdehnung definiert, wobei die Kondylusöffnung-Längsachse einen Mindestabstand zu der Knochenkontaktfläche des vorderen Rands aufweist, wobei der Mindestabstand mindestens dem Befestigungselementradius entspricht.

## Revendications

1. Composant fémoral de prothèse (31) de genou, comportant :
une bride (36) antérieure, ladite bride (36) antérieure présentant une surface en contact avec l'os de bride antérieure comportant une facette antérieure ; et
un condyle (40, 42) s'étendant depuis ladite bride (36) antérieure, ledit condyle présentant une surface (32) en contact avec l'os de condyle comportant une facette (58) (60) située à l'opposé de ladite facette (36) antérieure, ledit condyle (40, 42) présentant un alésage (62) de condyle formé à l'intérieur de ce dernier, ledit alésage (62) de condyle délimitant un axe (A2) longitudinal d'alésage de condyle qui recoupe ladite facette (58) (60) et ne recoupe pas ladite bride (36) antérieure.

2. Composant fémoral de prothèse de genou selon la revendication 1, dans lequel ledit axe (A2) longitudinal d'alésage de condyle recoupe ladite facette (58) (60) au niveau d'un point d'intersection, ledit point d'intersection étant positionné de sorte qu'un axe (P) perpendiculaire à ladite facette (58, 60) et passant par ledit point d'intersection a un espacement minimal par rapport à ladite bride antérieure (36) qui ne dépasse pas 8 mm.

3. Composant fémoral de prothèse de genou selon la revendication 1, dans lequel ledit axe (A2) longitudinal d'alésage de condyle recoupe ladite facette (58) (60) au niveau d'un point d'intersection, ledit point d'intersection étant positionné de sorte qu'un axe perpendiculaire à ladite facette (58) (60) et passant par ledit point d'intersection a un espacement minimal par rapport à ladite bride (36) antérieure qui ne dépasse pas 5 mm.

4. Composant fémoral de prothèse de genou selon la revendication 1, dans lequel ladite facette (58) (60) comporte une facette (58) chanfreinée postérieure.

5. Composant fémoral de prothèse de genou selon la revendication 1, dans lequel ladite facette (58, 60) comporte une facette (60) chanfreinée postérieure.

6. Composant fémoral de prothèse de genou selon la revendication 1, dans lequel ledit axe (A2) longitudinal d'alésage de condyle est à une distance minimale de 4 mm de ladite bride (36) antérieure.

7. Composant fémoral de prothèse de genou selon la revendication 1, dans lequel ledit axe (A2) longitudinal d'alésage de condyle est oblique par rapport à ladite surface (32) en contact avec l'os de condyle.

8. Prothèse fémorale de genou selon la revendication 1, dans laquelle ladite facette (58, 60) est orientée par rapport à ladite facette (36) antérieure de sorte que ledit axe (P) perpendiculaire à ladite facette (58, 60) et passant par ledit point d'intersection recoupe ladite facette (36) antérieure.

9. Composant fémoral de prothèse de genou selon la revendication 1, dans lequel l'axe (A2) longitudinal d'alésage de condyle forme un angle de 7 à 27° avec une ligne (P) perpendiculaire à la facette (58, 60).

10. Composant fémoral de prothèse de genou selon la revendication 1, dans lequel le composant fémoral de prothèse de genou comporte une surface (32) en contact avec l'os de prothèse fémorale de genou comportant la facette (36) antérieure et comportant en outre une facette (58) postérieure, une facette (54) chanfreinée antérieure, une facette (56) distale et une facette (60) chanfreinée postérieure, la facette (54) chanfreinée antérieure recouvrant la facette (36) antérieure et la facette (56) distale, la facette (60) chanfreinée postérieure recouvrant la facette (56) distale et la facette (58) postérieure.

11. Composant fémoral de prothèse de genou selon la revendication 4, dans lequel la facette postérieure diverge proximalement de la facette antérieure.

12. Composant fémoral de prothèse de genou selon la revendication 13, dans lequel un plan contenant la facette postérieure forme un angle de 1 à 5° avec un plan contenant la facette antérieure.

13. Composant fémoral de prothèse de genou selon la revendication 1, comportant en outre :
une augmentation (70) postérieure destinée à être fixée audit condyle (40, 42) ; et
un élément de fixation (90) servant à fixer ladite augmentation (70) postérieure audit condyle (40, 42).

14. Composant fémoral de prothèse de genou selon la revendication 13, dans lequel ladite augmentation (70) postérieure comprend un alésage d'augmentation formé à travers cette dernière, et dans lequel ledit élément de fixation (90) comporte un élément de fixation fileté, ledit élément de fixation (90) fileté étant dimensionné et formé de manière à pouvoir être aligné avec ledit axe (A2) longitudinal de l'alésage (62) de condyle et fixé par rapport à l'augmentation (70) postérieure et le composant fémoral de prothèse de genou en traversant ledit alésage d'augmentation et ledit alésage (62) de condyle pour mettre en prise ladite augmentation (70) et ledit condyle (40, 42), et fixer ladite augmentation (70) postérieure audit condyle (40, 42).

15. Composant fémoral de prothèse de genou selon la revendication 13, dans lequel ledit élément de fixation (90) délimite un axe longitudinal et une section périphérique et dans lequel ledit élément de fixation délimite en outre un rayon d'élément de fixation par rapport à ladite section périphérique, ledit axe longitudinal d'alésage de condyle étant à une distance minimale de ladite surface en contact avec l'os de bride antérieure, ladite distance minimale étant au moins égale audit rayon de l'élément de fixation.
